# EUROPEAN PATENT SPECIFICATION

(11) **EP 1 222 915 B1**
(45) Date of publication and mention of the grant of the patent: **07.09.2005**
(21) Application number: 02250257.9
(22) Date of filing: 15.01.2002
(51) Int. Cl.: A61K 7/48

(54) **Wet wiper impregnated with plant extracts**
Mit Pflanzenextrakten imprägniertes feuchtes Tuch
Lingette humide imprégnée d'un extrait à base de plantes

(30) Priority: 16.01.2001 JP 2001007826
(43) Date of publication of application: 17.07.2002
(73) Proprietor: UNI-CHARM CORPORATION, Ehime-ken (JP)
(72) Inventor: Miyazawa, Kiyoshi, c/o Technical Center, Mitoyo-gun, Kagawa-ken 769-1602 (JP); Hisanaka, Takayuki, c/o Technical Center, Mitoyo-gun, Kagawa-ken 769-1602 (JP)
(74) Representative: Fitchett, Stuart Paul

(56) References cited:
- EP-A- 0 350 275
- EP-A- 0 619 074
- WO-A-00/64408
- WO-A-00/65911
- WO-A-98/03147

## Description

### BACKGROUND OF THE INVENTION

### Field of the Invention

The present invention relates generally to a wet wiper for use in nursing patients or caring aged persons.

### Description of the Related Art

A wide variety of wet wipers for sanitary applications have been known in the art. For example, Japanese Unexamined Patent Publication No. 2000-191511 discloses wet paper which is impregnated with a solution containing plant-derived extracted chemicals, and to be used, particularly for women, to wipe external genitals and other insanitary parts. On the other hand, Japanese Unexamined Patent Publication No. 2000-226324 discloses a composition containing a liquid extract of plant for use in cleansing and cleaning applications and in cosmetic lotions.

However, since the wet paper described in Japanese Unexamined Patent Publication No. 2000-191511 is intended to be used for wiping women's private parts, it naturally has only small area. In addition, since it is made of paper, the basis weight is also small. Accordingly, this wet paper is not suitable for nursing or caring applications for wiping the entire body or buttocks of patients or aged parsons. Moreover, since it is essential to this wet paper that ethyl alcohol be impregnated thereto, the wet paper gives stimulation to the skin, particularly when used for wiping the skin of an aged person. With the ethyl alcohol contained, furthermore, a moisture retaining effect for skin being one feature of the plant-derived extracted chemicals cannot be utilized effectively.

On the other hand, Japanese Unexamined Patent Publication No. 2000-226324, the composition of which is constituted so as to be suitable also for use in cosmetic lotions, has no mention of applicability of the composition to nursing of patients and caring of aged persons. In addition, it has no mention of wipers suitable for nursing and caring.

Further, if a low alcohol such as ethyl alcohol is used for extraction of the liquid plant extract from plant, the low alcohol remains in the liquid chemical, resulting in stimulation to the skin, particularly, of aged persons, and reducing moisture retainability of the skin as described above.

### SUMMARY OF THE INVENTION

The present invention is worked out in view of the shortcomings in the prior art, and therefore, has an object to provide a wet wiper suitable for use in nursing patients and caring aged persons.

Another object of the present invention is to provide a wet wiper giving no stimulation to skin when used for wiping bodies of patients or aged persons, as well as capable of maintaining moisture retainability of skin and providing an anti-inflammatory effect and/or an effect of preventing eruption caused by enzymes in feces.

According to the present invention, there is provided a wet wiper comprising: a sheet including absorbent fibers; and a liquid impregnated into the sheet, the impregnated liquid contains at least one plant extract selected from the group consisting of: an extract of loquat leaves, an extract of peach leaves, a melilot extract, a chamomile extract, an aloe extract, a hibiscus extract, an extract of phellodendron bark or hinokitiol extract, characterise in that the alcohol ingredient used to extract the plant extract consists only of polyhydric alcohol.

The plant extract may have a moisture retaining effect, and may also have an anti-inflammatory effect and/or an inactivating effect for enzymes in feces.

The plant extract having an anti-inflammatory effect may be at least one selected from the group consisting of an extract of loquat leaves, an extract of peach leaves, a melilot extract, a chamomile extract, and an aloe extract; and the plant extract having an inactivating effect for enzymes in feces may be at least one selected from the group consisting of an extract of phellodendron barks, a hibiscus extract, and a hinokitiol extract.

Preferably, the polyhydric alcohol used for extracting the plant extract is at least one selected from the group consisting of 1,3-butylene glycol, propylene glycol, and glycerine.

Preferably, the impregnated liquid contains from 0.1% to 10% by weight liquid extract formed by extraction of the plant extract.

Preferably, the absorbent fibers comprise at least one of rayon and cotton, the sheet has a basis weight within a range of 40g/m² to 70g/m², and the sheet is rectangular in the shape and has a shorter side of 250 mm or more and an area of 1000 cm² or more.

Preferably, the wiper is for nursing and/or care use, and the sheet comprises a nonwoven fabric.

### PREFERRED EMBODIMENTS OF THE INVENTION

The wet wiper according to the present invention is directed to patients who require nursing and bed-ridden aged persons who require care.

According to one embodiment of the present invention, a wet wiper is formed from a relatively thick, soft, liquid-absorbent fibrous sheet (nonwoven fabric) comprising absorbent fibers, such as those of rayon, cotton or pulp. Among them, particularly preferred is rayon or cotton in view of softness. Of course, the sheet may comprise both of rayon and cotton. In addition to the absorbent fibers, the sheet further comprises synthetic fibers. The synthetic fibers may be mono-component synthetic fibers such as those of polypropylene, polyester (e.g., polyethylene terephthalate) or polyethylene. Alternatively, they may be bicomponent synthetic fibers, of which, for example, the core is polyester and the sheath is polyethylene, both the core and sheath are polyester, the core is polyester and the sheath is polypropylene, both the core and sheath are polypropylene, or the core is polypropylene and the sheath is polyethylene.

For the nonwoven fabric, a fibrous web may be formed either by dry-laid or wet-laid process. The individual fibers forming the fibrous web are preferably tied together by a spunlacing (hydroentangling) method to provide sufficient bulkiness and softness to the nonwoven fabric.

The blending ratio of the absorbent fibers is preferably within a range of 0.5% to 70% based on the total weight of the absorbent fibers and the synthetic fibers. When it is less than 0.5%, the nonwoven fabric becomes too stiff and cannot retain a sufficient amount of impregnating liquid. On the other hand, when it exceeds 70%, it becomes difficult to bring productivity and cost in good balance in the production of the nonwoven fabric.

The nonwoven fabric is preferably square or rectangular in the shape with a shorter side of 250 mm or more and an area of 1000 cm² or more. If smaller than the foregoing size, the wet wiper cannot certainly cover a single hand or both hands of a nursing person or carer, who intends to wipe the body of a patient or bed-ridden aged person with the wet wiper, so that the hand comes into direct contact with the body, thereby causing a safety and hygiene problem. In addition, a plurality of wet wipers are necessary for wiping the entire body and it also takes a much wiping time.

Further, the nonwoven fabric preferably has a basis weight in a range of 30g/m² to 70g/m². More preferably, the lower limit is equal to or more than 40g/m². When it is less than 30g/m², the sheet is excessively thin to give a trouble in wiping. On the other hand, when it exceeds 70g/m², it is difficult to handle with.

Since the wet wiper according to one embodiment of the present invention is formed from a nonwoven fabric with the size and the basis weight being in the foregoing ranges, it has size and thickness like those of a towel and is suitable for wiping the entire body or buttocks, particularly, of adult patients or aged persons

The nonwoven fabric according to one embodiment of the present invention is impregnated with a liquid containing a plant extract.

The plant extract includes one or more of an extract of phellodendron barks, a hibiscus extract, a hinokitiol extract, an extract of loquat leaves, an extract of peach leaves, a melilot extract, a chamomile extract and an aloe extract. The liquid may contain one or more of the above-named plant extracts. When the wet wiper containing at least one of the plant extracts is used for wiping the human skin, the skin can be provided with moisture retainability.

Further, when the liquid contains at least one of an extract of phellodendron barks, a hibiscus extract and a hinokitiol extract, as the plant extract, it can provide an effect of inhibiting the activity of lipase and protease which are enzymes contained in discharges (feces), preventing or suppressing diaper rash. When the liquid contains at least one of an extract of loquat leaves, an extract of peach leaves, a melilot extract, a chamomile extract and an aloe extract, on the other hand, it can provide an anti-inflammatory effect, preventing or suppressing skin roughness.

Of course, such liquid is also gentle to the skin of a nursing person or carer.

For example, about 1000 g of liquid extract of phellodendron barks can be obtained by adding 1200 g of 1,3-butylene glycol (1.3 BG) to 200g of barks of phellodendron excluding peripheral barks, conducting extraction at a room temperature for ten days under occasional stirring, and filtering it. In an alternative, about 900 g of liquid extract of loquat leaves can be obtained by adding 900 g of 1.3 BG and 100g of purified water to 100 g of powdered dried leaves of loquat, conducting extraction at a room temperature for ten days under occasional stirring, and filtering it.

Thereafter, a solvent such as propylene glycol or 1,3-butylene glycol is added to the liquid extract to form an impregnating liquid. Here, it is preferred that the impregnating liquid does not contain any lower alcohol such as ethyl alcohol, in view of preventing stimulation to the haman skin.

It is also possible to add an appropriate amount of antibacterial agent such as ethyl paraben or methyl paraben to the impregnating liquid for preventing growth of bacteria in cotton or rayon. The impregnating liquid may also contain an ingredient for providing odor-killing or deodorizing effect. Here, the impregnating liquid preferably contains the liquid extract in an amount of 0.1% to 10% by weight, more preferably 0.9% to 7% by weight, in view of the balance between the cost and effect.

The impregnating liquid is impregnated in an amount of 200% to 300% by weight of the nonwoven fabric. When the amount of the liquid is less than 200%, the liquid can not be impregnated efficiently to the thick nonwoven fabric entirely, so that no sufficient wiping effect can be provided and it can not fully take advantage of the moisture retaining effect, the anti-inflammatory effect and the inhibiting effect for enzymes in feces by the plant extract. On the contrary, when it is more than 300%, the liquid cannot be fully retained in the nonwoven fabric but may cause liquid dripping.

The wet wiper thus formed is usually folded into a compact size. A plurality of wet wipers thus folded are vertically stacked on each other and then accommodated in a sealable bag or casing. Then, upon use, the wet wipers are taken out one by one from the bag or the casing.

### [Example]

Examples, hereinafter set forth, describe the present invention in detail in comparison with Comparative Examples, but the present invention should not be restricted thereto.

### (Examples 1-4, Comparative Examples 1-4)

For Examples 1-4 and Comparative Examples 1, 2 and 4, used were spunlaced nonwoven fabrics, in which 15% by weight of polyester fibers, 30% by weight of polypropylene fibers and 55% by weight of rayon fibers were blended. The polyester fibers had a fineness of 1.3 dtex and a length of 44 mm; the polypropylene fibers had a fineness of 1.7 dtex and a length of 45 mm; and the rayon fibers had a fineness of 1.4 dtex and a length of 44 mm. For Comparative Example 3, used was a spunlaced nonwoven fabric, in which polyester fibers and polypropylene fibers were blended in an amount of 50% by weight, respectively. The sheet sizes and basis weights (g/m²) were shown in Table 1.

Then, liquids to be impregnated into the nonwoven fabrics were prepared to contain the liquid plant extracts in the amounts shown in Table 1, 5% by weight of propylene glycol, 0.15% by weight of methyl paraben, 0.1% by weight of ethyl paraben and the balance of ion exchanged water to make up 100% by weight in total. The impregnating liquid was impregnated in an amount of 250% by weight of the nonwoven fabric.

In Table 1, the liquid plant extracts A, B and C were prepared from phellodendron barks, loquat leaves and aloe, respectively. As described above, the plant extract A was obtained as about 1000 g of plant extract by adding 1200g of 1,3-butylene glycol (1.3 BG) to 200 g of barks of phellodendron excluding peripheral barks, conducting extraction at a room temperature for 10 days while occasionally stirring, and then separating extract by filtration. The plant extract B was obtained as about 900 g of plant extract by adding 900g of 1.3 BG and 100g of purified water to 100g of powdered dry leaves of loquat, conducting extraction at a room temperature for 10 days while occasionally stirring, and then separating extract by filtration.

Further, the plant extract C was obtained as 1000g of plant extract by adding 550 g of 1,3-butylene glycol and 550 g of purified water to 50g of powder formed by filtering, purifying and drying.a juice of leaves of aloe vera and variations thereof, stirring for five hours, standing still at a room temperature for 10 days, and then filtering the extract.

The plant extract D was obtained as about 1000 g of plant extract by adding 1200 g of a liquid mixture of ethanol (ethyl alcohol) and purified water (7:3) to 200 g of barks of phellodendron or species of same genus excluding peripheral barks, conducting extraction at a room temperature for 10 days while occasionally stirring, and then filtering the extract.

Here, the content for each of the plant extracts A, B and C in Table 1 means the content (% by weight) of the liquid extract, which was obtained by extracting plant extract with 1,3-butylene glycol, or 1.3 BG and purified water, in the impregnating liquid. The content for the plant extract D means the content (% by weight) of the liquid extract, which was obtained by extracting the plant extract with ethyl alcohol and purified water, in the impregnating liquid.

### (Evaluation Method)

A monitor assuming a carer (hereinafter called " care giving monitor") wiped the entire bodies of ten monitors assuming bed-ridden aged persons (hereinafter called " care receiving monitors") with the wet wipers of Examples 1-4 and Comparative Examples 1-4. Then, the care giving monitor was asked whether wiping could be conducted effectively or not. On the other hand, the care receiving monitors were asked, whether they felt any pain or discomfort during wiping by the care giving monitor. In Table 1, with respect to easiness of wiping, the symbol "○" represents the case where all the carve giving and care receiving monitors felt favorable; the symbol "×" represents the case where one or more monitors felt not favorable.

Further, the skin conditions of the care receiving monitors were visually observed just after wiping. Then, the skin conditions were again visually observed three hours after wiping. In Table 1, the symbol "○" represents the case where no particular roughness or redness of skin was recognized; the symbol " ×" represents the case where roughness or redness was recognized.

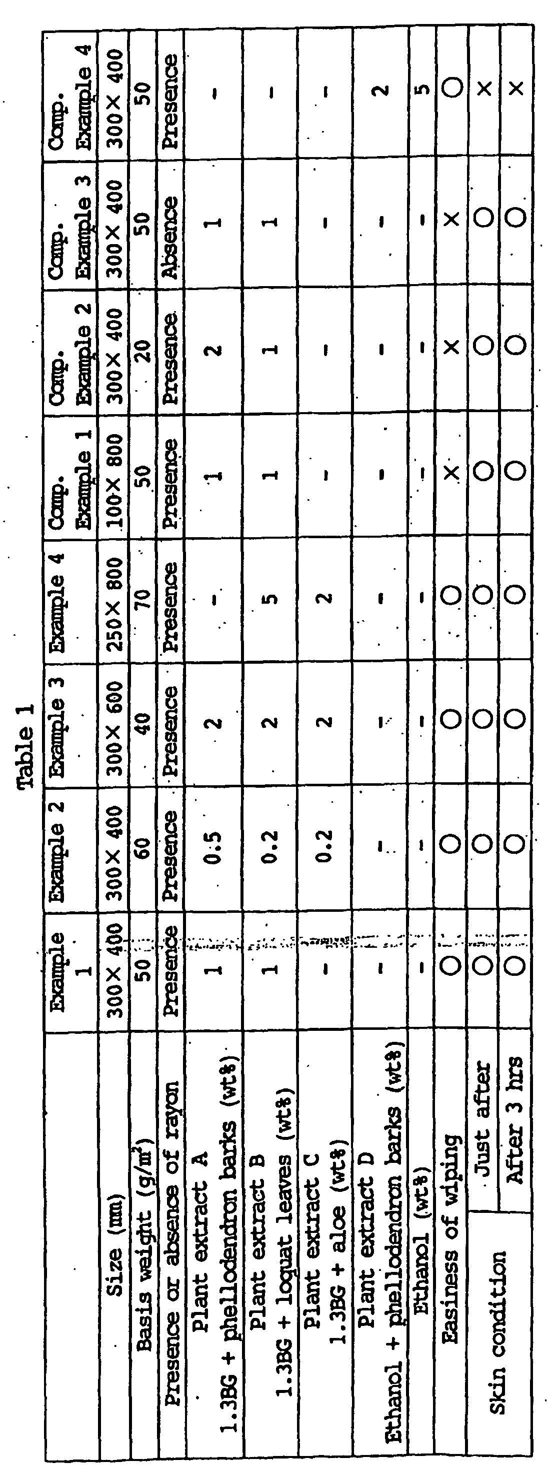

As shown in the results of Table 1, Comparative Example 1 could not provide smooth wiping as the sheet being too small in size; Comparative Example 2 could not provide smooth wiping as the sheet being too thin; Comparative Example 3 could not provide smooth wiping as the impregnating liquid could not be sufficiently retained in the sheet not containing absorbent fibers; and Comparative Example 4 resulted in confirmation of deterioration of skin condition both just after and three hours after wiping as the plant extract extracted with the polyhydric alcohol was not contained therein. On the contrary, in Examples 1 to 4, since the size and the thickness of the sheet were sufficient, and the sheet contained the absorbent rayon and the plant extracts extracted with the polyhydric alcohol, satisfactory results could be obtained regarding both easiness of wiping and effects on skin.

The present invention should not be understood as limited to the specific embodiment set out above but to include all embodiments within the scope of the appended claims.

## Claims

1. A wet wiper comprising: a sheet including absorbent fibers; and a liquid impregnated into the sheet, the impregnated liquid contains at least one plant extract selected from the group consisting of: an extract of loquat leaves, an extract of peach leaves, a melilot extract, a chamomile extract, an aloe extract, a hibiscus extract, an extract of phellodendron bark or hinokitiol extract, **characterised in that**
the alcohol ingredient used to extract the plant extract consists only of polyhydric alcohol.

2. A wet wiper as set forth in claim 1, wherein the plant extract has a moisture retaining effect.

3. A wet wiper as set forth in claim 1, wherein the plant extract has an anti-inflammatory effect.

4. A wet wiper as set forth in claim 1, wherein the plant extract has an inactivating effect for enzymes in feces.

5. A wet wiper as set forth in claim 3, wherein the plant extract is at least one selected from the group consisting of an extract of loquat leaves, an extract of peach leaves, a melilot extract, a chamomile extract, and an aloe extract.

6. A wet wiper as set forth in claim 4, wherein the plant extract is at least one selected from the group consisting of an extract of phellodendron barks, a hibiscus extract, and a hinokitiol.

7. A wet wiper as set forth in claim 1, wherein the impregnated liquid contains only polyhydric alcohol as alcoholic ingredient.

8. A wet wiper as set forth in claim 1, wherein the polyhydric alcohol used extracting the plant extract is at least one selected from the group consisting of 1,3-butylene glycol, propylene glycol, and glycerin.

9. A wet wiper as set forth in claim 1, wherein the impregnated liquid contains from 0.1% to 10% by weight liquid extract formed by extraction of the plant extract.

10. A wet wiper as set forth in claim 1, wherein the absorbent fibers comprise at least one of rayon and cotton.

11. A wet wiper as set forth in claim 1, wherein the sheet has a basis weight within a range of 40g/m² to 70g/m².

12. A wet wiper as set forth in claim 1, wherein the sheet is rectangular in the shape and has a shorter side of 250 mm or more and an area of 1000 cm² or more.

13. A wet wiper as set forth in claim 1, wherein the wiper is for nursing and/or care use.

14. A wet wiper as set forth in claim 1, wherein the sheet comprises a nonwoven fabric.

## Patentansprüche

1. Feuchttuch, aufweisend: ein Blatt mit absorbierenden Fasern; und eine Flüssigkeit, mit der das Blatt imprägniert ist und die mindestens einen Pflanzenextrakt enthält, der aus folgender Gruppe ausgewählt ist: Japanmispelblattextrakt, Pfirsichblattextrakt, Steinklee-Extrakt, Kamillenextrakt, Aloe-Extrakt, Hibiskusextrakt, Phellodendron-Rindenextrakt oder Hinokitiolextrakt, **dadurch gekennzeichnet, daß**
der zur Extraktion des Pflanzenextrakts verwendete Alkoholbestandteil lediglich aus mehrwertigem Alkohol besteht.

2. Feuchttuch nach Anspruch 1, wobei der Pflanzenextrakt eine feuchtigkeitsbewahrende Wirkung aufweist.

3. Feuchttuch nach Anspruch 1, wobei der Pflanzenextrakt eine entzündungshemmende Wirkung aufweist.

4. Feuchttuch nach Anspruch 1, wobei der Pflanzenextrakt eine inaktivierende Wirkung für Enzyme in Fäkalien aufweist.

5. Feuchttuch nach Anspruch 3, wobei der Pflanzenextrakt mindestens ein aus der aus Japanmispelblattextrakt, Pfirsichblattextrakt, Steinklee-Extrakt, Kamillenextrakt und Aloe-Extrakt bestehenden Gruppe ausgewählter Extrakt ist.

6. Feuchttuch nach Anspruch 3, wobei der Pflanzenextrakt mindestens ein aus der aus Phellodendron-Rindenextrakt, Hibiskusextrakt und Hinokitiolextrakt bestehenden Gruppe ausgewählter Extrakt ist.

7. Feuchttuch nach Anspruch 1, wobei die Imprägnierflüssigkeit als Alkoholbestandteil lediglich mehrwertigen Alkohol enthält.

8. Feuchttuch nach Anspruch 1, wobei der zur Extraktion des Pflanzenextrakts verwendete mehrwertige Alkohol mindestens ein aus der aus 1,3-Butylenglykol, Propylenglykol und Glyzerin bestehenden Gruppe ausgewählter Alkohol ist.

9. Feuchttuch nach Anspruch 1, wobei die Imprägnierflüssigkeit von 0,1 bis 10 Gew-% Flüssigextrakt enthält, der durch Extraktion des Pflanzenextrakts gebildet ist.

10. Feuchttuch nach Anspruch 1, wobei die absorbierenden Fasern Reyon und/oder Baumwolle enthalten.

11. Feuchttuch nach Anspruch 1, wobei das Blatt ein Basisgewicht im Bereich von 40 g/m² bis 70 g/m² aufweist.

12. Feuchttuch nach Anspruch 1, wobei das Blatt eine rechteckige Form und eine kurze Seite von 250 mm oder mehr und eine Fläche von 1000 cm² oder mehr aufweist.

13. Feuchttuch nach Anspruch 1 zur Verwendung bei der Pflege und/oder Betreuung.

14. Feuchttuch nach Anspruch 1, wobei das Blatt ein Vlies enthält.

## Revendications

1. Lingette humide comprenant : une feuille comprenant des fibres absorbantes ; et un liquide imprégné dans la feuille, le liquide imprégné contenant au moins un extrait de plantes choisi dans le groupe constitué par : un extrait de feuilles de néflier du Japon, un extrait de feuilles de pêcher, un extrait de mélilot, un extrait de camomille, un extrait d'aloès, un extrait d'hibiscus, un extrait d'écorces de philodendron ou un extrait d'hinokitiol, **caractérisée en ce que**
l'ingrédient alcoolisé pour extraire l'extrait de plantes est constitué exclusivement par de l'alcool polyhydrique.

2. Lingette humide selon la revendication 1, dans laquelle l'extrait de plantes possède un effet de rétention de l'humidité.

3. Lingette humide selon la revendication 1, dans laquelle l'extrait de plantes possède un effet anti-inflammatoire.

4. Lingette humide selon la revendication 1, dans laquelle l'extrait de plantes possède un effet inactivant pour les enzymes dans les fèces.

5. Lingette humide selon la revendication 3, dans laquelle l'extrait de plantes est au moins l'un choisi dans le groupe constitué par un extrait de feuilles de néflier du Japon, un extrait de feuilles de pêcher, un extrait de mélilot, un extrait de camomille, et un extrait d'aloès.

6. Lingette humide selon la revendication 4, dans laquelle l'extrait de plantes est au moins l'un choisi dans le groupe constitué par un extrait d'écorces de philodendron, un extrait d'hibiscus, et un hinokitiol.

7. Lingette humide selon la revendication 1, dans laquelle le liquide d'imprégnation contient seulement de l'alcool polyhydrique comme ingrédient alcoolisé.

8. Lingette humide selon la revendication 1, dans laquelle l'alcool polyhydrique utilisé pour extraire l'extrait de plantes est choisi dans le groupe constitué par le glycol 1,3-butylène, le glycol propylène et la glycérine.

9. Lingette humide selon la revendication 1, dans laquelle le liquide d'imprégnation contient de 0,1 % à 10 % en poids d'un extrait liquide formé par extraction de l'extrait de plantes.

10. Lingette humide selon la revendication 1, dans laquelle les fibres absorbantes contiennent au moins soit de la rayonne, soit du coton.

11. Lingette humide selon la revendication 1, dans laquelle la feuille a un poids de base dans une plage de 40 g/m² à 70 g/m².

12. Lingette humide selon la revendication 1, dans laquelle la feuille est de forme rectangulaire et a un petit côté égal ou supérieur à 250 mm, et une surface égale ou supérieure à 1000 cm².

13. Lingette humide selon la revendication 1, dans laquelle la lingette est destinée aux soins infirmiers et/ou aux traitements.

14. Lingette humide selon la revendication 1, dans laquelle la feuille comprend une étoffe non tissée.
